# FASCICULE DE BREVET EUROPEEN

(11) **EP 0 758 550 B1**
(45) Date de publication et mention de la délivrance du brevet: **31.03.1999**
(21) Numéro de dépôt: 96401734.7
(22) Date de dépôt: 05.08.1996
(51) Int. Cl.: A61L 27/00

(54) **Procédé de fabrication d'un produit comprenant du carbonate de calcium et de la chaux pour revêtement de prothèse**
Verfahren zur Herstellung eines Materials enthaltend Calziumcarbonat und Kalk zur Beschichtung von Prothesen
Method of producing a material containing calcium carbonate and lime for coating prostheses

(30) Priorité: 14.08.1995 FR 9509865; 14.11.1995 FR 9513578
(43) Date de publication de la demande: 19.02.1997
(73) Titulaire: TEROLAB SERVICES - SNMC, 94290 Villeneuve-Le-Roi (FR)
(72) Inventeur: Lahille, Michel, 91430 Vauhallan (FR); Ben-Mokhtar, Mourad, 75006 Paris (FR)
(74) Mandataire: Cabinet Martinet & Lapoux

(56) Documents cités:
- EP-A- 0 022 724
- EP-A- 0 212 929
- EP-A- 0 523 372
- WO-A-87/07826
- WO-A-94/17838
- WO-A-94/26322
- LA RECHERCHE, vol. 25, no. 262, Février 1994, PARIS, FR, pages 208-210, XP000420760 E. LOPEZ, S. BERLAND AND A. LE FAOU: "LA NACRE AU SERVICE DU SQUELETTE HUMAIN"

## Description

La présente invention concerne un produit comprenant du carbonate de calcium et biocompatible pour revêtir au moins partiellement une prothèse afin d'intégrer celle-ci dans le milieu osseux ambiant.

Des produits contenant du carbonate de calcium sont préconisés par CAMPRASSE Georges dans les brevets et demandes de brevet FR-B-2 647 334, FR-A-2 706 308 et WO-A-94/17838 pour fabriquer notamment des pièces profilées, telles qu'implants et prothèses en chirurgie orthopédique, ou des racines dentaires de substitution. Ces produits sont fabriqués à partir de nacre issue de mollusques aquatiques, par exemple de la nacre de Pinctada Maxima, après avoir subi un traitement spécifique, mécanique, thermique et chimique. Ce traitement spécifique consiste à faire subir à la partie nacrée obtenue préalablement par une préparation mécanique de coquilles de mollusque, des opérations physico-chimiques de trempage, lavage, rinçage, traitements par vapeur d'eau notamment en présence d'agents chimiques, et enfin de séchage par paliers thermiques.

En particulier, des produits de CAMPRASSE peuvent être utilisés sous forme de poudre et possèdent des propriétés adhésives qui rendent les produits aptes à être associés à d'autres matériaux, tels que des métaux ou des polymères organiques, afin de fournir un ciment biologique de scellement pour toutes prothèses. Les produits ainsi obtenus peuvent être utilisés comme enduit sur certains métaux, et non comme revêtement intégré à ceux-ci, pour en augmenter l'adhésion avec le tissu osseux.

Les produits de CAMPRASSE peuvent également être utilisés sous forme compacte pour réaliser à eux seuls une prothèse, comme par exemple un plateau tibial, un condyle fémoral, une prothèse de hanche, une vis, etc.

Les propriétés biocompatibles des produits de CAMPRASSE résultent principalement de la composition à base de carbonate de calcium sous la forme à la fois de calcite et d'aragonite. La présence de carbonate de calcium dans le squelette de coraux madréporaires, tels que les Porites, a également incité l'usage de ceux-ci notamment en chirurgie dentaire.

Les produits de CAMPRASSE offrent principalement deux inconvénients :
- leur origine animale les rend très difficiles, voir inaptes, à une homologation dans certains pays ;
- leur coût est élevé à cause de la partie nacrée dont ils sont principalement issus, qui est un matériau relativement rare, et à cause du matériel et du personnel nécessaires aux opérations physicochimiques particulières de fabrication.

La présente invention vise à fournir un procédé de fabrication d'un produit pour revêtement de prothèse qui est entièrement d'origine minérale, moins coûteux et au moins aussi histocompatible que les produits selon la technique antérieure ci-dessus.

A cette fin, un procédé de fabrication d'un produit pour revêtement de prothèse est tel que défini dans la revendication 1.

Ce produit constituant un revêtement sur la prothèse est complètement intégré à son support, tel que prothèse, et est donc distinct et non équivalent à un enduit utilisé par CAMPRASSE, qui par sa nature pâteuse est apposée sur la prothèse et séparable de celle-ci.

D'autres caracteristiques du procédé de fabrication sont énoncées dans les revendications 2 à 12.

Le produit de revêtement selon l'invention ne comprend aucune matière d'origine organique, particulièrement animale, lorsqu'il est apposé sur la prothèse. En effet, dans le produit selon l'invention, le carbonate de calcium peut être sous la forme de calcite, de préférence au moins jusqu'à 75 % de la masse du produit, et/ou d'aragonite, de préférence au moins jusqu'à 25 % de la masse du produit, et de la chaux peut être en proportion d'au plus 60 % de la masse du produit, la chaux étant composée de chaux anhydre CaO et/ou de chaux hydratée Ca(OH)₂. Selon une autre variante, la chaux dans le produit de revêtement peut comprendre de la chaux hydratée Ca(OH)₂, de préférence en proportion d'au plus 25 % de la masse du produit. Par exemple, le produit de revêtement comprend 65 % à 75 % de calcite avec quelques traces d'aragonite, inférieure à 10 % de la masse du produit, et 25 % à 35 % de chaux anhydre avec quelques traces de chaux hydratée, inférieure à 10 % de la masse du produit.

Le produit selon l'invention est histocompatible. L'examen radiologique montre que le produit selon l'invention contribue à la croissance de tissu osseux dur autour de la prothèse, si bien que quelques mois après l'implantation prothétique, le produit n'apparaît plus distinctement du milieu osseux environnant. La prothèse est complètement entourée de tissu osseux, contrairement au tissu fibreux développé généralement autour d'une prothèse revêtue d'hydroxyapatite.

Plus particulièrement, la projection thermique est appliquée à :
- soit un composé initial comprenant essentiellement, c'est-à-dire dans une proportion d'au moins 95 % à 100 % de la masse du composé l'un des composants suivants : aragonite, calcite, chaux anhydre et chaux hydratée,
- soit un composé initial comprenant au moins l'un des composants aragonite et calcite, dans une large proportion pouvant varier de 10 à 90 % de la masse du composé, et le cas échéant de la chaux, c'est-à-dire de la chaux anhydre CaO et de la chaux anydre hydratée Ca(OH)₂), dans une plus faible proportion, typiquement de 5 à 50 % de la masse du composé.

A titre d'exemple, les compositions avec les proportions de composant suivantes ont donné des résultats satisfaisants :
- aragonite en proportion de 30 à 90 % de la masse du composé et calcite en proportion de 10 à 70 % de la masse du composé ;
- aragonite en proportion de 50 à 90 % de la masse du composé et chaux anhydre CaO en proportion de 10 à 50 % de la masse du composé ;
- calcite en proportion de 50 à 90 % de la masse du composé et chaux anhydre CaO en proportion de 10 à 50 % de la masse du composé ;
- aragonite en proportion de 20 à 80 % de la masse du composé, calcite en proportion de 10 à 70 % de la masse du composé, et chaux anhydre CaO en proportion de 10 à 50 % de la masse du composé ;
- aragonite en proportion de 20 à 80 % de la masse du composé, calcite en proportion de 10 à 70 % de la masse du composé, et chaux hydratée Ca(OH)₂ en proportion de 10 à 50 % de la masse du composé ;
- aragonite en proportion de 80 à 95 % de la masse du composé et chaux hydratée Ca(OH)₂ en proportion de 5 à 20 % de la masse du composé ; et
- calcite en proportion de 80 à 95 % de la masse du composé et chaux hydratée Ca(OH)₂ en proportion de 5 à 20 % de la masse du composé.

Les quatre composants principaux précités, savoir l'aragonite, la calcite, la chaux anhydre et la chaux hydratée, entrant ensemble dans au moins 50 % environ du composé sont tous d'origine minérale, ce qui réduit considérablement le coût du composé brut pour fabriquer le produit selon l'invention, comparativement à la nacre d'origine animale préconisée pour fabriquer le produit de CAMPRASSE. La réduction de coût est d'environ 80 à 90 %. En effet, la calcite qui est le carbonate rhomboédrique de calcium, dit également spath d'Islande, est très répandue à l'état naturel dans les marbres, la craie, etc ; il en est de même de l'aragonite, variété cristalline orthorhombique du carbonate de calcium. La chaux, qu'elle soit sous forme de chaux anhydre CaO, dite également chaux vive, ou qu'elle soit sous forme de chaux hydratée Ca(OH)₂, dite également chaux éteinte ou Portlandite, est un composé commercial courant produit en grande quantité.

La projection thermique du composé est produite à des températures voisines de la température de fusion du calcium égale à 839 °C, c'est-à-dire à des températures comprises entre 750 °C et 900 °C de sorte qu'une proportion de calcite se transforme en chaux avec dégagement de gaz carbonique et, le cas échéant, en plus faible proportion en aragonite qui elle-même se transforme au moins partiellement en calcite à des températures intermédiaires de 480°C environ. Lorsque de la chaux hydratée Ca(HO)₂ est prévue dans le composé initial, celle-ci est transformée en chaux anhydre CaO par élimination d'eau lors du chauffage du composé. Pour ces raisons, en général, la proportion de calcite dans le composé initial est inférieure à celle dans le produit final de revêtement prothétique selon l'invention, et la proportion de chaux anhydre, lorsqu'elle existe, dans le composé initial peut être inférieure à celle dans le produit final de revêtement prothétique.

Les diverses proportions des composants dans le produit final de revêtement dépendent notamment des composants sélectionnés et de leurs porportions dans le produit initial, ainsi que des caractéristiques mécaniques du produit initial et des caractéristiques du traitement thermique du composé.

A titre d'exemple, les composés et proportions particulières de composants suivants peuvent être sélectionnés pour former un composé à partir duquel un produit de revêtement selon l'invention est obtenu :
1) aragonite et calcite respectivement en proportion de 30 et 70 %, ou 90 % et 10 % ;
2) 50 % d'aragonite ou de calcite avec 50 % de la chaux anhydre CaO, ou 90 % d'aragonite ou de calcite avec 10 % de chaux anhydre ;
3) 80 % d'aragonite, 10 % de calcite et 10 % de chaux anhydre ; ou bien 20 % d'aragonite, 70 % de calcite et 10 % de chaux anhydre ; ou bien 30 % d'aragonite, 20 % de calcite et 50 % de chaux anhydre ;
4) 90 % d'aragonite ou de calcite et 10 % de chaux hydratée Ca(OH)₂.

Pour certaines applications, le composé initial peut être enrichi d'alumine Al₂O₃, de préférence en proportion de 5 à 50 % de la masse du composé.

La projection thermique est obtenue au moyen d'une torche à plasma dans laquelle le composé est introduit sous forme pulvérulente dans le jet de plasma, ou dans lequel des composants du composé sont introduits séparément sous forme pulvérulente dans le jet de plasma.

D'autres caractéristiques et avantages de la présente invention apparaîtront plus clairement à la lecture de la description suivante de plusieurs réalisations préférées de l'invention en référence aux dessins annexés correspondants dans lesquels :
- la figure 1 montre schématiquement, à titre d'exemple non limitatif, une installation de revêtement de prothèse par projection thermique avec une tête de torche à plasma avec injection externe de poudre de composé selon une première réalisation, pour la mise en oeuvre du procédé de fabrication du produit selon l'invention ; et
- la figure 2 montre une vue en coupe détaillée de la tête d'une torche à plasma avec injections internes séparées de poudres de composant, selon une seconde réalisation du procédé.

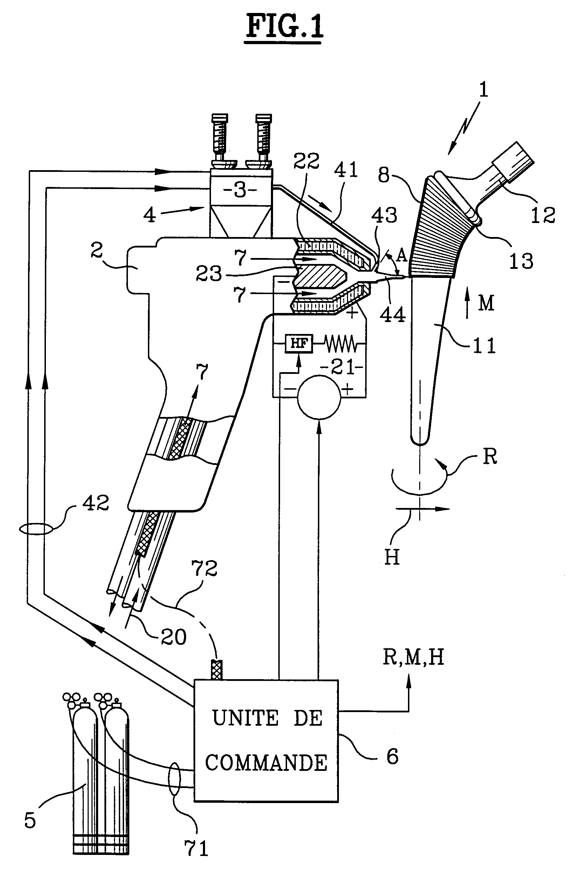

La projection thermique du composé sur une prothèse 1, telle que la queue fémorale d'une prothèse de hanche, est une projection plasmagène atmosphérique obtenue au moyen d'une torche à plasma 2 refroidie par eau 20, comme montré à la figure 1. Dans la torche, le composé sous forme pulvérulente 3 est injecté depuis un distributeur de poudre 4 à travers un conduit de poudre sous pression 41 dans un jet de plasma fortement exothermique 44 et acquiert une énergie cinétique élevée pour être projeté et déposé sur la surface de la prothèse 1. La torche à plasma 2 est reliée à un réservoir de bouteilles 5 équipé de débitmètre et contenant des gaz, tel qu'argon et/ou azote, ainsi que d'autres gaz additifs tels qu'hydrogène et/ou hélium. Le mélange gazeux peut être un mélange de :
argon et hydrogène, ou
azote et hélium, ou
argon et azote, ou
argon, hydrogène et hélium.

Une unité de commande 6 est reliée par des canalisations 71 au réservoir 5 pour régler les proportions des gaz et leur débit en un mélange gazeux prédéterminé 7 amené à la torche par une conduite 72.

L'unité de commande 6 également contrôle des sources de tension notamment à haute fréquence 21 de la torche 2 et asservit également le débit du composé pulvérulent 3 dans le distributeur de poudre 4 à travers des liaisons électriques et pneumatiques 42 en fonction du dépôt de produit de revêtement souhaité.

Selon la réalisation montrée à la figure 1, l'injection de poudre est externe à une buse anodique 22. En tête de la torche 2, devant la buse anodique 22 que traverse un gaz ionisé à haute température issu du mélange gazeux 7, débouche une extrémité d'injection de poudre 43 d'un conduit de poudre 41. L'extrémité d'injection 43 du conduit de poudre 41 est de préférence orientée vers l'intérieur de la torche 2, en sens opposé au jet de plasma, et inclinée d'un angle A compris entre 45° et 90° environ. Grâce à cette inclinaison, le composé pulvérulent pénètre plus profondément et centralement dans le jet de plasma 44 et est mieux réparti au centre de celui-ci, ce qui uniformise le traitement thermique des grains de poudre.

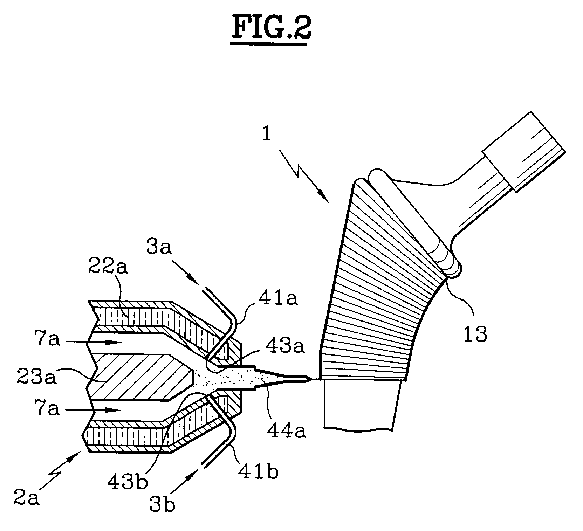

Selon une autre variante montrée à la figure 2, au lieu que l'injection de poudre 1 s'effectue de manière externe, l'injection de poudre est interne à l'anode. L'orifice d'injection 43a d'un conduit de poudre 41a débouche devant la buse anodique 22a à l'intérieur de la torche 2a, devant la cathode 23a et dans le conduit d'injection de mélange gazeux 7a. Cette variante offre les avantages d'augmenter la concentration de poudre dans les zones chaudes centrales du jet de plasma 44a et d'entraîner tous les grains de poudre à la vitesse du jet de plasma.

La morphologie, la pureté et la granulométrie de la poudre injectée dans le jet de plasma sont sélectionnées en fonction des caractéristiques du produit de revêtement souhaité et du rendement de projection.

Les poudres des composants constituant le composé initial pulvérulent ne sont pas utilisables dans leur état brut de livraison. Le rendement de la projection thermique est d'autant plus amélioré que les granules composant les poudres sont pures, sphéroïdales, et fines. Initialement, les poudres des composants sont dépurées de produits organiques et autres impuretés. Cette purification est effectuée par un traitement thermique sur les poudres, typiquement de 300 à 400 °C, avant l'injection des poudres, ou bien par leur passage ultérieur dans le jet de plasma.

La sphéroïdisation est de préférence réalisée par une atomisation des poudres. Elle consiste à préparer un mélange des poudres sous forme de barbotine, et puis à atomiser les granules de la barbotine en des particules de poudre de forme sphérique. La barbotine est une masse pâteuse relativement liquide résultant du mélange des poudres avec de l'eau distillée, des floculants pour éviter des agglomérats de poudre, et un liant tel que l'alcool polyvinylique. La poudre de composé ainsi obtenue présente une granulométrie de préférence comprise entre 10 et 250 µm.

Lorsque plusieurs poudres composent le composé à injecter, les poudres peuvent être mélangées :
- soit avant atomisation, le mélange étant ensuite atomisé et injecté par un unique conduit 41 comme décrit ci-dessus et illustré à la figure 1 ;
- soit après atomisations séparées des poudres des composants en des masses pâteuses 3a, 3b, ... et injections séparées des masses pâteuses par des orifices d'injection respectifs 43a, 43b, ... de conduits 41a, 41b, ... reliés à des bacs d'alimentation respectifs, comme illustré à titre d'exemple dans la figure 2, les granules injectés étant alors mélangés par passage dans le jet de plasma 44a.

Selon la réalisation illustrée à la figure 1, la torche à plasma est fixe et la sortie de tête de la torche dirige le jet de plasma horizontal 44 vers la surface de prothèse à recouvrir par le produit 8 selon l'invention. La torche à plasma 2 fonctionne avec un arc interne, dit également arc transféré, c'est-à-dire aucun courant n'est conduit par le jet de plasma 44 à l'extérieur devant la buse anodique 22. Selon une autre variante, la torche à plasma fonctionne avec un arc externe et le courant parcourt entièrement le jet de plasma jusqu'à la prothèse 2 à revêtir qui est polarisée positivement et joue le rôle d'anode externe.

Comme montré schématiquement à la figure 1, la prothèse 1 est montée sur un mandrin d'un plateau. Le plateau est de préférence celui d'une table micrométrique qui anime la prothèse d'un déplacement prédéterminé dans l'espace à trois dimensions, par exemple comprenant en combinaison une rotation R autour d'un axe vertical, une translation montante M et une translation horizontale H. Ce déplacement prédéterminé R, M, H de la prothèse par rapport à la torche à plasma, ou le déplacement de la torche à plasma par rapport à la prothèse qui est fixe ou seulement animée selon un ou deux déplacements R, M, H selon d'autres variantes, est obtenu par une programmation de l'unité de commande 6 qui commande des moteurs de la table micrométrique afin que le jet de plasma 44 balaye la surface de la prothèse sur laquelle le produit 8 selon l'invention doit être déposé. La tête de la torche 2 est placée à quelques centimètres de la prothèse 1 afin que les particules de poudre soient déposées superficiellement sur la prothèse à une température qui est comprise entre 750 °C à 950 °C environ, sélectionnée en fonction des compositions et proportions choisies du mélange pulvérulent et du mélange plasmagène et donc de la composition du dépôt du produit 8 finalement retenue.

Selon la réalisation illustrée, la prothèse 1 est une queue de prothèse de hanche dont la majeure partie, la tige prothétique 11, doit être revêtue par le produit de revêtement 8 selon l'invention. La partie proximale supérieure de la prothèse de hanche constituée principalement d'un col 12 destiné à recevoir un cotyle prothétique et d'une collerette 13 sont épargnés du dépôt de produit.

Le produit de revêtement peut être déposé en une seule couche de 10 µm à 100 µm d'épaisseur, en tournant la prothèse autour d'un axe vertical et en montant (ou descendant) et en translatant horizontalement progressivement la prothèse. La durée de dépôt d'une telle prothèse est de l'ordre de quelques minutes pour déposer 10 à 20 grammes de produit de revêtement 8 selon l'invention.

En fonction du type de prothèse et d'exigences réglementaires notamment en matière de contamination, l'opération de projection et dépôt peut être effectuée dans une enceinte sous vide ou dans une enceinte sous atmosphère contrôlée. A la place d'une torche à plasma, la projection thermique peut être effectuée avec un pistolet à tir discontinu, dit canon à détonation, dans lequel les composants pulvérulents ou le composé pulvérulent est introduit dans une chambre de gaz de combustion et est éjectée périodiquement par explosions.

Bien que la prothèse illustrée à la figure 1 soit une prothèse de hanche, le produit de revêtement selon l'invention peut être déposé thermiquement sur d'autres prothèses et implants métalliques ou céramiques, notamment en titane ou alumine, pour faciliter la croissance osseuse. Ces prothèses et implants peuvent être, à titre non limitatif, des vis, tiges et plaques d'ostéosynthèse, des cages ou cales intervertébrales, des coins d'ostéotomie, des tiges ou queues et/ou cotyles prothètiques pour articulations telles que hanche, genou, épaule, doigt. Le produit de prothèse peut être déposé aussi bien en surface externe de ces prothèses ou implants que sur les parois internes de trous ou cavités ménagés dans les prothèses et implants.

## Revendications

1. Procédé de fabrication d'un produit comprenant du carbonate de calcium et de la chaux pour revêtement de prothèse caractérisé par la projection thermique au moyen d'un jet de plasma (44), d'un composé comprenant au moins l'un des composants suivants : aragonite, calcite, chaux anhydre et chaux hydratée, pour revêtir au moins partiellement une prothèse (1) avec ledit produit (8).

2. Procédé conforme à la revendication 1, selon lequel le jet de plasma est obtenu au moyen d'une torche à plasma (2, 2a) dans laquelle ledit composé (3) est introduit sous forme pulvérulente dans le jet de plasma (44), ou des composants (3a, 3b) du composé sont introduits séparément sous forme pulvérulente dans le jet de plasma (44a).

3. Procédé conforme à la revendication 2, selon lequel le composé sous forme pulvérulente ou les composants du composé sous forme pulvérulente sont injectés sensiblement en direction opposée au jet de plasma (44 ; 44a) avec une inclinaison (A) comprise entre 45° et 90° environ par rapport au jet de plasma.

4. Procédé conforme à l'une quelconque des revendications 1 à 3, selon lequel le jet de plasma (44 ; 44a) est constitué par l'un de gaz plasmagènes (7, 7a) suivants comprenant de l'argon et de l'hydrogène ; de l'azote et de l'hélium ; de l'argon et de l'azote ; et de l'argon, de l'hydrogène et de l'hélium.

5. Procédé conforme à l'une quelconque des revendications 1 à 4, comprenant le déplacement contrôlé (R, D, H) de la prothèse (1) devant le jet de plasma (44).

6. Procédé conforme à l'une quelconque des revendications 1 à 5, selon lequel le produit (8) revêtant la prothèse (1) a une épaisseur inférieure à 100 µm environ, de préférence comprise entre 10 µm et 100 µm.

7. Procédé conforme à l'une quelconque des revendications 1 à 6, selon lequel ledit produit (8), lorsqu'il est en train de revêtir la prothèse (1), a une température comprise entre 750 et 900°C environ.

8. Procédé conforme à l'une quelconque des revendications 1 à 7, selon lequel le composé comprend en outre de l'alumine (Al₂0₃), de préférence en proportion de 5 à 50 % de la masse du composé.

9. Procédé conforme à l'une quelconque des revendications 1 à 8, selon lequel le composé est une poudre (3) ayant une granulométrie de préférence comprise entre 10 et 250 µm.

10. Procédé conforme à l'une quelconque des revendications 1 à 9, comprenant préalablement une atomisation de poudres de composant mélangées sous forme de masse pâteuse (3), et une injection (43) de la masse pâteuse dans le jet de plasma (44).

11. Procédé conforme à l'une quelconque des revendications 1 à 10, comprenant préalablement des atomisations séparées de poudres de composant, en des masses pâteuses (3a, 3b), et des injections séparées (43a, 43b) des masses pâteuses dans un jet gazeux (44a).

12. Procédé conforme à la revendication 10 ou 11, comprenant préalablement à toute atomisation de poudre de composant une purification de celle-ci.

## Claims

1. A method of manufacturing a product comprising calcium carbonate and lime for covering a prosthesis, characterized by thermal sputtering by means of a plasma jet (44) a substance comprising at least one of the following constituents: aragonite, calcite, quick lime and hydrated lime for covering at least partially a prosthesis (1) with said product (8).

2. A method according to claim 1, wherein the plasma jet is obtained by means of a plasma torch (2, 2a) in which said substance (3) is introduced in powder form into the plasma jet (44), or constituents (3a, 3b) of the substance are introduced separately in powder form into the plasma jet (44a).

3. A method according to claim 2, wherein the substance in powder form or the constituents of the substance in powder form are injected in substantially the opposite direction to the plasma jet (44 ; 44a) with an inclination (A) between about 45° and about 90° relative to the plasma jet.

4. A method according to any one of claims 1 to 3, wherein the plasma jet (44 ; 44a) is made up of one of following plasma-generating gases (7, 7a) comprising argon and hydrogen; nitrogen and helium; argon and nitrogen; and argon, hydrogen and helium.

5. A method according to any one of claims 1 to 4, comprising the controlled displacement (R, D, H) of the prosthesis (1) in front of the plasma jet (44).

6. A method according to any one of claims 1 to 5, wherein the product (8) covering the prosthesis (1) has a thickness less than about 100 µm, preferably lying between 10 µm and 100 µm.

7. A method according to any one of claims 1 to 6, wherein when covering the prosthesis (1), said product (8) is at a temperature lying between about 750° C and about 900° C.

8. A method according to any one of claims 1 to 7, wherein the substance further comprises alumina (Al₂0₃), preferably 5% to 50% by weight of the substance.

9. A method according to any one of claims 1 to 8, according which the substance is a powder (3) having a particle size preferably lying between 10 and 250 µm.

10. A method according to any one of claims 1 to 9, comprising beforehand atomizing powders of mixed constituents in the form of a pasty mass (3), and injecting (43) the pasty mass into the plasma jet (44).

11. A method according to any one of claims 1 to 10, comprising beforehand separately atomizing powders of constituents in pasty masses (3a, 3b), and separately injecting (43a, 43b) the pasty masses into a gas jet (44a).

12. A method according to claim 10 or 11, comprising prior to atomizing the powder of constituent purifying thereof.

## Patentansprüche

1. Verfahren zur Herstellung eines Kalziumkarbonat und Kalk enthaltenden Materials zur Beschichtung von Prothesen, gekennzeichnet durch die thermische Aufspritzung mit Hilfe eines Plasmastrahls (44) eines Gemisches, das mindestens einen der folgenden Bestandteile enthält: Aragonit, Kalzit, Branntkalk, Löschkalk, um mindestens teilweise eine Prothese (1) mit dem genannten Material (8) zu beschichten.

2. Verfahren nach Patentanspruch 1, dem zufolge der Plasmastrahl mit Hilfe eines Plasmabrenners (2, 2a) erhalten wird, in den das genannte Gemisch (3) pulverförmig in den Plasmastrahl (44) eingeführt wird oder Bestandteile (3a, 3b) des Gemisches getrennt pulverförmig in den Plasmastrahl (44a) eingeführt werden.

3. Verfahren nach Patentanspruch 2, dem zufolge das pulverförmige Gemisch oder die pulverförmigen Bestandteile des Gemisches im wesentlichen in der dem Plasmastrahl (44; 44a) entgegengesetzten Richtung mit einer Neigung (A) zwischen ungefähr 45° und 90° relativ zum Plasmastrahl eingespritzt werden.

4. Verfahren nach irgendeinem der Patentansprüche 1 bis 3, dem zufolge der Plasmastrahl (44; 44a) mit einem der folgenden plasmagenen Gase (7, 7a) erzeugt wird, die Argon und Wasserstoff, Stickstoff und Helium, Argon und Stickstoff, und Wasserstoff und Helium umfassen.

5. Verfahren nach irgendeinem der Patentansprüche 1 bis 4, die gesteuerte Verschiebung (R, D, H) der Prothese (1) vor dem Plasmastrahl (44) umfassend.

6. Verfahren nach irgendeinem der Patentansprüche 1 bis 5, dem zufolge das Material (8), mit dem die Prothese (1) beschichtet ist, eine Dicke von weniger als ungefähr 100 µm aufweist, vorzugsweise von zwischen 10 µm und 100 µm.

7. Verfahren nach irgendeinem der Patentansprüche 1 bis 6, dem zufolge das genannte Material (8), wenn die Prothese (1) mit ihm beschichtet wird, eine Temperatur hat, die zwischen ungefähr 750 und 900°C liegt.

8. Verfahren nach irgendeinem der Patentansprüche 1 bis 7, dem zufolge das Gemisch ausserdem Aluminiumoxid (Al₂O₃) enthält, vorzugsweise in einem Anteil von 5 bis 50% der Masse des Gemisches.

9. Verfahren nach irgendeinem der Patentansprüche 1 bis 8, dem zufolge das Gemisch ein Pulver (3) mit einer Korngrösse ist, die vorzugsweise zwischen 10 und 250 µm liegt.

10. Verfahren nach irgendeinem der Patentansprüche 1 bis 9, vorher das Zerstäuben von gemischten Pulvern von Bestandteilen in Form einer teigartigen Masse (3) umfassend und eine Einspritzung (43) der teigartigen Masse in den Plasmastrahl (44).

11. Verfahren nach irgendeinem der Patentansprüche 1 bis 10, vorher das getrennte Zerstäuben von Pulvern von Bestandteilen in Form teigartiger Massen (3a, 3b) umfasst und getrennte Einspritzungen (43a, 43b) der teigartigen Massen in einen Gasstrahl (44a).

12. Verfahren nach Patentanspruch 10 oder 11, vor jeglichem Zerstäuben von Pulvern von Bestandteilen deren Reinigung umfassend.
